# EUROPEAN PATENT APPLICATION

(11) **EP 3 569 172 A1**
(43) Date of publication of application: **20.11.2019**
(21) Application number: 18739060.4
(22) Date of filing: 10.01.2018
(51) Int. Cl.: A61B 18/12, A61B 18/08, A61B 18/14, A61B 18/18

(54) **PUNCTURE DEVICE AND CARTRIDGE FOR PUNCTURE DEVICE**

(30) Priority: 11.01.2017 JP 2017002736
(71) Applicant: Saeki, Masanori, Tamano-shi, Okayama 706-0224 (JP)
(72) Inventor: Saeki, Masanori, Tamano-shi, Okayama 706-0224 (JP)
(74) Representative: Hoffmann Eitle
(86) International application number: PCT/JP2018/000386
(87) International publication number: WO 2018/131623

(57) **Abstract**

Provided is a puncture device capable of suppressing excessive heating and insufficient heating of a living body by understanding to what degree the living body is being heated. A puncture device 1 according to one embodiment of the present invention includes a puncture needle 10 that punctures a puncture site of the living body and supplies thermal energy, a sensor needle 11 having a puncture site temperature sensor 81 that measures the temperature of the puncture site, a holder 12 that holds the puncture needle 10 and the sensor needle 11, a cartridge 60 that receives the puncture needle 10, the sensor needle 11, and the holder 12, a main body 30 to which the cartridge 60 is attachable, a contacting unit 20 provided with through holes 28 penetrating the front and back surfaces thereof and provided on the surface side thereof with a contacting surface 24b with the surface of the puncture site, and a drive unit 40 that moves the holder 12 such that the puncture needle 10 and the sensor needle 11 are projected and retracted from the contacting surface 24b via the through holes 28.

## Description

### Field

The present invention relates to a puncture device including a puncture needle that punctures a living body and a cartridge for the puncture device.

### Background

In the related art, there has been disclosed a puncture device that includes an electrode needle and a contacting unit provided with a through hole into which the electrode needle can be inserted, and allows the contacting unit to be brought into close contact with the epidermis surface of a living body so that the electrode needle is projected (see Patent Literature 1). Due to the destruction of sweat glands by such a puncture device, for example, it is possible to suppress excessive secretion of sweat from apocrine glands or eccrine glands.

### Citation List

### Patent Literature

Patent Literature 1: International Publication No. 2015-055323

### Summary

### Technical Problem

Sweat glands, which are destruction targets of the puncture device, are present at various depth positions from the dermis to the subcutaneous tissue. The thickness of the dermis or the subcutaneous tissue changes depending on the site of the living body as well as individual differences. Therefore, in order to reliably destroy the sweat glands, it is necessary to heat the living body over the entire depth direction toward the subcutaneous tissue from the dermis while gradually changing the puncture depth of the electrode needle in accordance with the thickness of the dermis or the subcutaneous tissue.

However, in the related puncture device, since it is not possible to understand to what degree the living body (including the dermis and the subcutaneous tissue (the same applies below)) is being heated by the electrode, the living body may be excessively heated or may be insufficiently heated.

The present invention has been made to solve the aforementioned problems, and an object of the present invention is to provide a puncture device capable of suppressing excessive heating or insufficient heating of a living body by understanding to what degree the living body is being heated, and a cartridge for the puncture device.

### Solution to Problem

A puncture device according to an aspect of the present invention includes a puncture needle configured to puncture a puncture site of a living body and supply energy to the puncture site; a holder configured to hold the puncture needle; a cartridge configured to receive the holder so as to be slidable and having an opening through which the puncture needle is projected; a main body to which the cartridge is detachably attached; a contacting unit having a through hole through which the puncture needle is projected to the puncture site of the living body and having a cooling member that is able to come into contact with the puncture site; a sensor needle in which a temperature sensor is arranged; and a control unit. The sensor needle is installed in parallel with the puncture needle and is held by the holder so as to project to the puncture site of the living body via the opening formed in the cartridge and the through hole formed in the contacting unit, and the control unit is configured to control the energy supplied to the puncture needle in accordance with output of the temperature sensor.

Furthermore, in the puncture device according to an aspect of the present invention, the control unit can be configured to supply the energy to the puncture needle such that an output temperature of the temperature sensor is 50°C or more and 150°C or less.

Furthermore, in the puncture device according to an aspect of the present invention, the control unit can be configured to supply an alternating current to the puncture needle.

Furthermore, in the puncture device according to an aspect of the present invention, the control unit can be configured to supply energy to the puncture needle for a prescribed time. In the puncture device according to an aspect, the prescribed time can be a time between one and 20 seconds.

Furthermore, in the puncture device according to an aspect of the present invention, a surface of the puncture needle can be coated with an insulating material in a state in which a tip side of the puncture needle is exposed. Furthermore, in the puncture device according to an aspect, an exposure length of the tip side of the puncture needle can be 0.1 to 2.0 mm. Furthermore, in the puncture device according to an aspect, the insulating material can be made by glass coating or resin coating.

Furthermore, in the puncture device according to an aspect of the present invention, a surface temperature sensor can be provided on a side of the contacting unit, the side facing the puncture site, to measure a temperature of a surface of the puncture site, and the control unit can be configured to control a temperature of the cooling member in accordance with the output of the surface temperature sensor.

Moreover, a cartridge for a puncture device according to another aspect of the present invention includes a puncture needle configured to puncture a puncture site of a living body and supply energy to the puncture site; a holder configured to hold the puncture needle; a cartridge configured to receive the holder so as to be slidable and having an opening through which the puncture needle is projected; and a sensor needle in which a temperature sensor is arranged. The sensor needle is installed in parallel with the puncture needle and is held by the holder so as to project via an opening formed in the cartridge.

Furthermore, in the cartridge for the puncture device according to another aspect of the present invention, a surface of the puncture needle can be coated with an insulating material in a state in which a tip side of the puncture needle is exposed. Furthermore, in the cartridge for the puncture device according to an aspect, an exposure length of the tip side of the puncture needle can be 0.1 to 2.0 mm. Furthermore, in the cartridge for the puncture device according to an aspect, the insulating material can be made by glass coating or resin coating.

### Advantageous Effects of Invention

According to the puncture device according to an aspect of the present invention and the cartridge for the puncture device according to another aspect of the present invention, it is possible to accurately understand to what degree a puncture site of a living body is being heated, to suppress the puncture site from being excessively heated or insufficiently heated, and to safely and reliably destroy sweat glands.

### Brief Description of Drawings

Fig. 1 is a view illustrating a puncture device according to one embodiment according to the present invention.
Fig. 2 is a side view of the puncture device according to the embodiment according to the present invention.
Fig. 3 is a top view of the puncture device according to the embodiment according to the present invention.
Fig. 4 is a view illustrating a contacting unit of the puncture device according to the embodiment according to the present invention.
Fig. 5A is a sectional view taken along line VA-VA of Fig. 2, and Fig. 5B is a schematic sectional view of a puncture site sensor of Fig. 5A.
Fig. 6 is a view illustrating a state before a cartridge is attached to a main body of the puncture device according to the embodiment according to the present invention.
Fig. 7 is a view illustrating a state in which a drive unit of the puncture device according to the embodiment according to the present invention is operated.
Fig. 8 is a view illustrating a state before a puncture needle of the puncture device according to the embodiment according to the present invention is projected.
Fig. 9 is a view illustrating a state in which the puncture needle of the puncture device according to the embodiment according to the present invention is projected.
Fig. 10 is a view illustrating a state in which the puncture needle of the puncture device according to the embodiment according to the present invention has reached a membrane.
Fig. 11A is a graph illustrating change over time of a tip position of the puncture needle when the puncture device according to the embodiment of the present invention is operated, and Fig. 11B is a graph illustrating change over time of a temperature detected by a sensor needle in a similar manner.
Fig. 12A illustrates a temperature distribution measurement result at a predetermined point of time when an RF current flows through the puncture device according to the embodiment of the present invention, without using a cooling plate 24, and Fig. 12B illustrates a temperature distribution measurement result at a predetermined point of time when the RF current flows through the puncture device according to the embodiment of the present invention in a similar manner, by using the cooling plate 24.

### Description of Embodiments

Hereinafter, with reference to the drawings, a puncture device 1 according to an embodiment of the present invention and a cartridge 60 for the puncture device will be described. It is noted that the following embodiment exemplifies the puncture device 1 and the cartridge 60 for the puncture device for embodying the technical scope of the present invention, does not specify the present invention to the puncture device 1 and the cartridge 60 for the puncture device, and can be equally applied to other embodiments included in the scope of the claims.

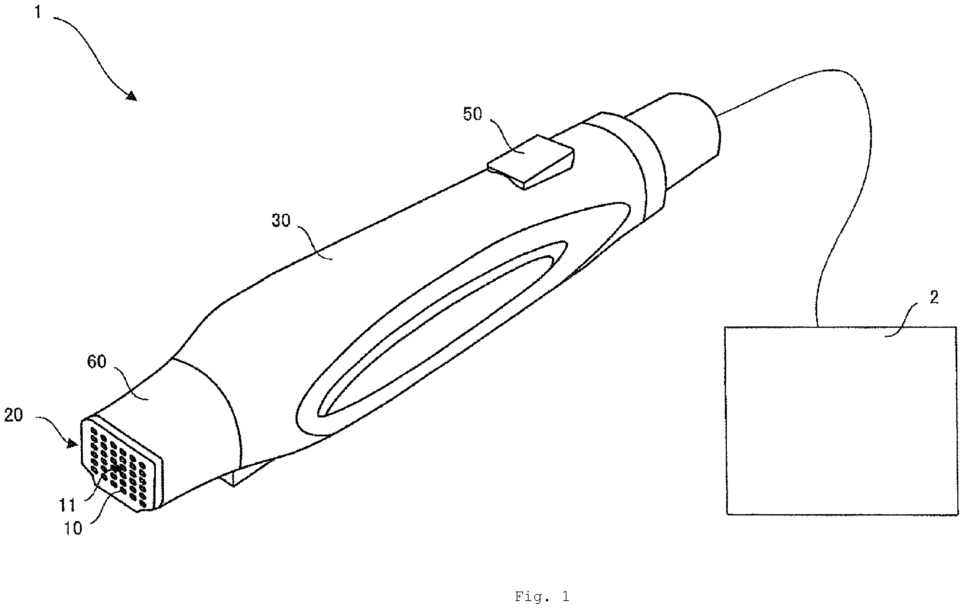

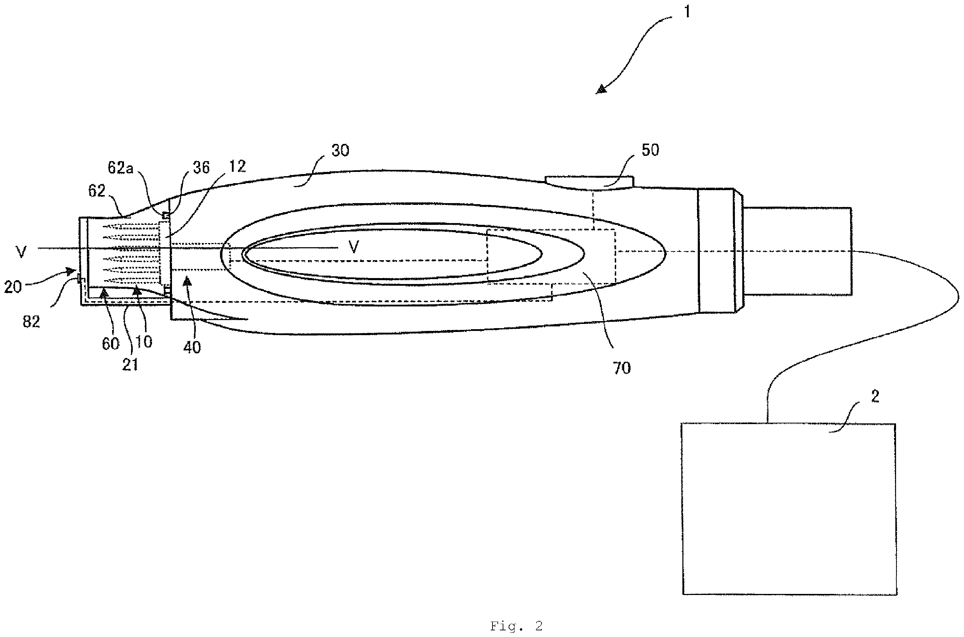

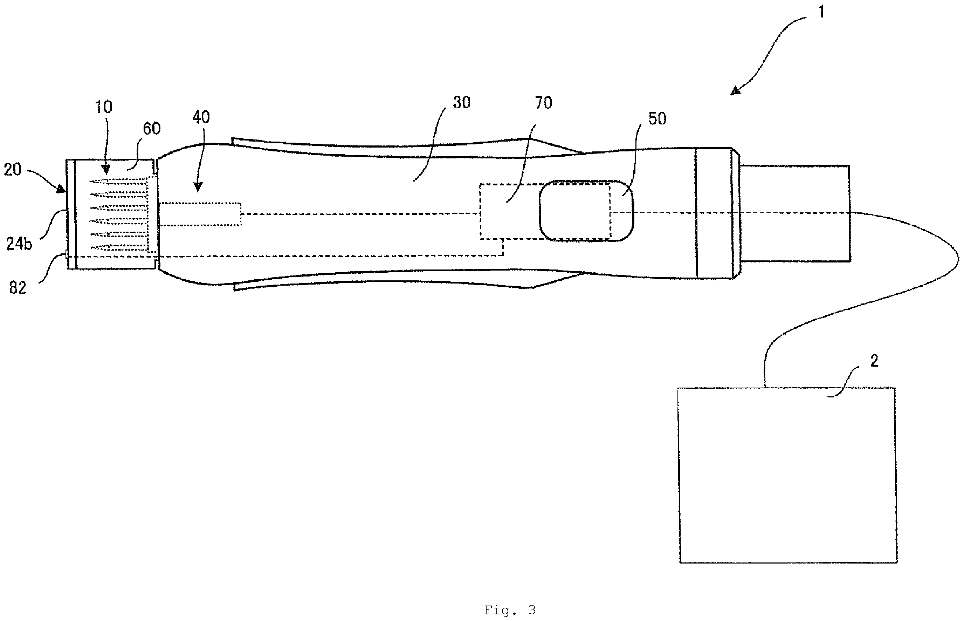

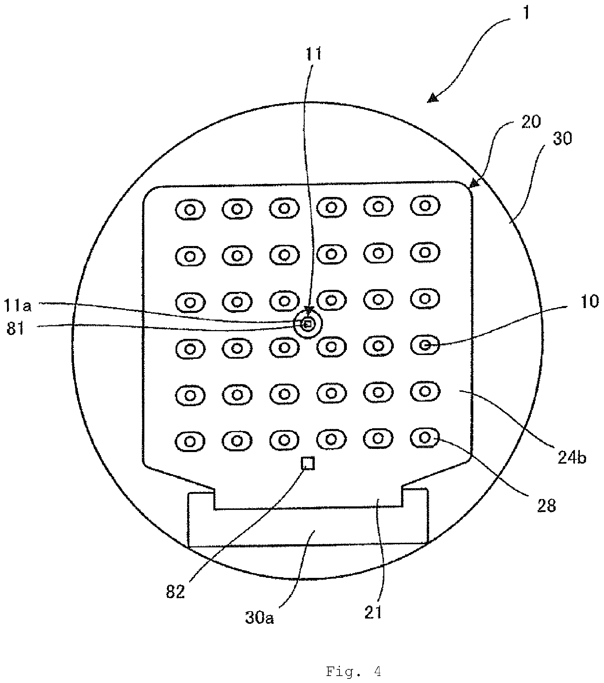

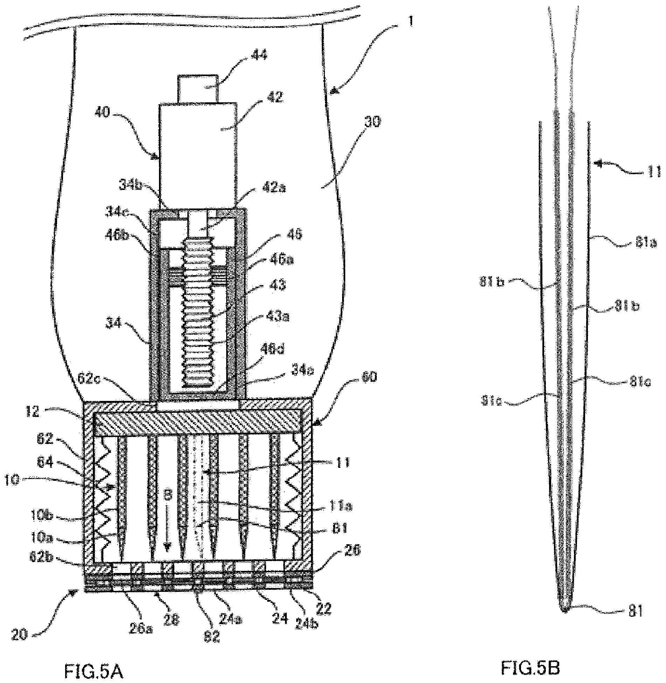

The puncture device 1 according to the present embodiment will be described using Fig. 1 to Fig. 5. Fig. 1 is a view illustrating the puncture device 1 according to the embodiment according to the present invention. Fig. 2 is a side view of the puncture device 1 according to the embodiment according to the present invention. Fig. 3 is a top view of the puncture device 1 according to the embodiment according to the present invention. Fig. 4 is a view illustrating a contacting unit 20 of the puncture device 1 according to the embodiment according to the present invention. Fig. 5A is a sectional view taken along line V-V of Fig. 2 and Fig. 5B is a schematic sectional view of a puncture site sensor of Fig. 5A.

The puncture device 1 according to the present embodiment includes at least a puncture needle 10 that punctures a puncture site of a living body, a sensor needle 11 that measures the temperature of the puncture site of the living body, a contacting unit 20 that comes into contact with the puncture site, a drive unit 40 that drives the puncture needle 10, a main body 30 that receives the drive unit 40, an operating unit 50 that operates the drive unit 40, and the cartridge 60. In the present invention, the puncture site of the living body is a part of the living body that can be punctured by the puncture needle 10. The puncture device 1 according to the present embodiment punctures the puncture site including sweat glands by using the puncture needle 10 and supplies thermal energy by the supply of energy from an energy supply unit 2, thereby destroying the sweat glands and suppressing excessive sweating.

The puncture needle 10, for example, is made of a conductive metal material such as a stainless steel. A tip part 10a of the puncture needle 10 is tapered so as to be puncturable. In the puncture needle 10, a part other than the tip part 10a is covered with an insulating film 10b made of an electrically insulating material. In order to safely and reliably destroy the sweat glands, it is preferable to expose the tip part 10a and cover the part other than the tip part 10a with the insulating film 10b. However, in the puncture needle 10, a part to be covered with the insulating film 10b is not necessarily the part other than the tip part 10a and it is sufficient if the puncture needle 10 includes a part made of a conductive metal material and a part in which the metal material is covered with the insulating film 10b made of an electrically insulating material. Furthermore, when the puncture needle 10 is not covered with the insulating film 10b, safety is reduced, but it is possible to supply the puncture site including the sweat glands with the thermal energy by the supply of energy from the energy supply unit 2.

The exposure length of the tip part 10a, for example, can be set to about 0.1 to 2.0 mm or about 0.1 to 1.5 mm. When the exposure length of the tip part 10a is as short as 0.1 to 0.2 mm, accurate machining is difficult. Furthermore, when the exposure length of the tip part 10a is as long as 0.6 mm or more, since no current is concentrated on the tip part 10a, temperature is reduced. More preferably, the exposure length of the tip part 10a is about 0.2 to 0.6 mm. This is determined from the viewpoint that the thermal energy by the supply of energy from the energy supply unit 2 is supplied to the puncture site including the sweat glands without excess or deficiency.

For the insulating film 10b, glass coating is preferable from the viewpoint that an adhesion property is good and good insulating property can be ensured even though the thickness is made thin; however, the present invention is not limited thereto and for example, resin coating or coating using other materials may be used and it is preferable that coating be difficult to peel off. The thickness of the glass coating is not particularly limited, but for example, can be set to 10 to 25 µm, 12 to 18 µm, for example, 25 to 40 µm, for example, or 10 µm or less. When the thickness is made thick, since the ambient temperature of the glass coating does not rise, it is preferable in terms of burn prevention and suppression of the formation of a scab. When the diameter of the puncture needle 10 can be made small, the thickness of the coating can be set to about 25 to 100 µm, for example. In such a case, it is possible to suppress the diameter of a hole to be formed in the skin from becoming large. These are determined from the viewpoint that the thermal energy by the supply of energy from the energy supply unit 2 is supplied to the puncture site including the sweat glands without excess or deficiency.

The puncture needle 10 is arranged in a plural number in a matrix form and is fixed to a rectangular plate-like holder 12 made of a plastic material and the like, for example. The number of puncture needles 10 is not particularly limited and may be one. When the puncture needle 10 is arranged in a plural number, it is preferable to arrange about 20 to 40 needles (or more), for example, and an interval between adjacent puncture needles 10 is set to about 0.5 to 3 mm (more preferably, 1 to 3 mm), for example. The thickness of each puncture needle 10 is preferably about 0.1 to 0.3 mm, for example. The arrangement shape of the puncture needles 10 may be various shapes, such as an annular shape and a polygonal shape, in addition to the matrix shape. These are arranged or set from the viewpoint that the thermal energy by the supply of energy from the energy supply unit 2 is supplied to the puncture site including the sweat glands without excess or deficiency.

The tip part 10a of the puncture needle 10 may be formed in the shape of a blunt needle (a non-beveled needle having no blade surface on the tip thereof) that penetrates the skin (epidermis) and the subcutaneous tissue of the living body, but does not penetrate a membrane tissue part between the subcutaneous tissue and a muscle layer. With this, it is possible to safely puncture only a depth area where the sweat glands are likely to be present. It is preferable that the insertion depth of the puncture needle 10 be adjustable between 1.0 to 8.0 mm because an optimal insertion depth differs depending on patients.

As illustrated in Fig. 5B, the sensor needle 11 has a structure in which a temperature sensor 81 is attached to an internal closed end side of a hollow needle 11a to measure the temperature of the puncture site. The hollow needle 11a includes, for example, a SUS (stainless steel) hollow needle 81a having a closed end portion, and has an outer surface subjected to gold plating in order to obtain good thermal conduction and a surface provided with glass coating for insulation (all are not illustrated). The temperature sensor 81 herein uses an extremely fine thermocouple and its output is transmitted to a control unit 70 by an extremely fine lead wire 81c provided with an insulating coating 81b. The sensor needle 11 is installed in parallel with the puncture needle 10 in an empty space of the holder 12 where the puncture needle 10 is not attached. The sensor needle 11 is formed to have the same length as that of the puncture needle 10 and moves in the same manner as the puncture needle 10. Although one sensor needle 11 is provided in the present embodiment, when a plurality of sensor needles 11 are installed, it is possible to more finely measure the temperature distribution of the puncture site.

The contacting unit 20 includes a cooling part capable of cooling the puncture site and the vicinity of the puncture site, and includes a Peltier element 22, a cooling plate 24, and a heat dissipation block 26. The Peltier element 22 has a well-known configuration in which a p-type semiconductor and an n-type semiconductor are thermally arranged in parallel, the cooling plate 24 is provided on a heat absorption side of the Peltier element 22, and the heat dissipation block 26 is provided on a heat generation side of the Peltier element 22. The Peltier element 22 is arranged with an appropriate size in a plural number in a matrix form. The cooling plate 24 and the heat dissipation block 26 are provided with a plurality of openings 24a and 26a in gaps between the Peltier elements 22, and a plurality of through holes 28 penetrating the front and back surfaces of the contacting unit 20 are formed by the openings 24a and 26a facing each other.

The cooling plate 24 has a planar-like contacting surface 24b on the surface side thereof, can bring the contacting surface 24b into close contact with the epidermis and the like of a living body or a human body (hereinafter, simply referred to as "epidermis and the like"), and for example, has a sectional L shape or T shape (the shape of a surface part contacting with the epidermis and the like). The contacting surface 24b may have a shape that can be brought into close contact with the epidermis and the like, in addition to the planar shape, and for example, can be formed in a curved surface shape of an arc shape or a wave shape. The cooling plate 24 is provided with the opening 24a through which the puncture needle 10 penetrates, and it is not possible to directly cool the surface of the epidermis and the like at the part of the opening 24a. Therefore, a scab is formed on the epidermis and the like for each opening 24a, that is, at each interval (0.5 to 3 mm) between adjacent puncture needles 10. This cooling plate 24 is provided to cool the surface of the skin after heating, or before and after heating, so that it is possible to reduce a burn. Particularly, by using the cooling plate 24 having the sectional L shape or T shape, it is possible to improve a cooling effect of the surface of the skin.

In the case of using the cooling plate 24 having the sectional L shape or T shape, the contacting surface 24b of the cooling plate 24 has an area projected to be larger than an area provided with the opening 24a through which the puncture needle 10 penetrates, so that it is possible to simultaneously cool areas other than the surface area of the actually heated epidermis and the like (corresponding to the area provided with the opening 24a through which the puncture needle 10 penetrates). Accordingly, when paying attention to the area of the epidermis and the like to be heated, the area can be cooled in an area where the contacting surface 24b is projected after heating (can be cooled simultaneously with heating of another area) and can also be cooled in advance in the area where the contacting surface 24b is projected before heating (can be cooled simultaneously with the heating of another area). In the case of employing a configuration of injecting cooling gas to the opening 24a, it is possible to cool the surface of the epidermis and the like corresponding to the opening 24a even during heating.

The cooling part may have a configuration embedded with a cooling pipe through which refrigerant passes, and the like, for example, in addition to the cooling configuration using energization as described above. Moreover, the cooling part may have a cooling configuration of injecting cooling gas toward the epidermis and the like, for example, in addition to the configuration of cooling the epidermis and the like by being brought into close contact with the epidermis and the like as described above. In such a case, a surface temperature sensor 82 is attached to the contacting surface 24b and a temperature measured by the surface temperature sensor 82 is transmitted to the control unit 70. Furthermore, also in the cooling configuration using energization as described above, the surface temperature sensor 82 may be attached to the contacting surface 24b and a temperature measured by the surface temperature sensor 82 may also be transmitted to the control unit 70.

The through holes 28 may have various shapes such as a honeycomb shape, a mesh shape, and a slit shape. The through holes 28 formed in the contacting unit 20 do not necessarily need to correspond to the puncture needles 10 and the sensor needles 11 in a one-to-one manner, and the puncture needles 10 or a combination of the puncture needle 10 and the sensor needle 11 may be inserted into one through hole 28. In such a case, the through hole 28 may be formed in the contacting unit 20 in the form of an elongated hole, for example, and in order to allow two puncture needles 10 or two needles of one puncture needle 10 and one sensor needle 11 to pass through both end edge parts of the through hole 28 in a longitudinal direction, each puncture needle 10 or the sensor needle 11 may be fixed to the holder 12.

With such a configuration, it is possible to facilitate the insertion of each puncture needle 10 or the sensor needle 11 into the through hole 28 and the inner peripheral surface of the through hole 28 can guide the advance and retraction of each puncture needle 10 or the sensor needle 11, so that it is possible to more reliably puncture the surface of the epidermis and the like (hereinafter, simply referred to as "epidermis surface"). The contacting unit 20 does not necessarily have a function of cooling the puncture site and it is sufficient if it is possible to come into contact with the puncture site, but, as already mentioned, it is better to provide the cooling function in order to reduce a burn of the surface of the skin.

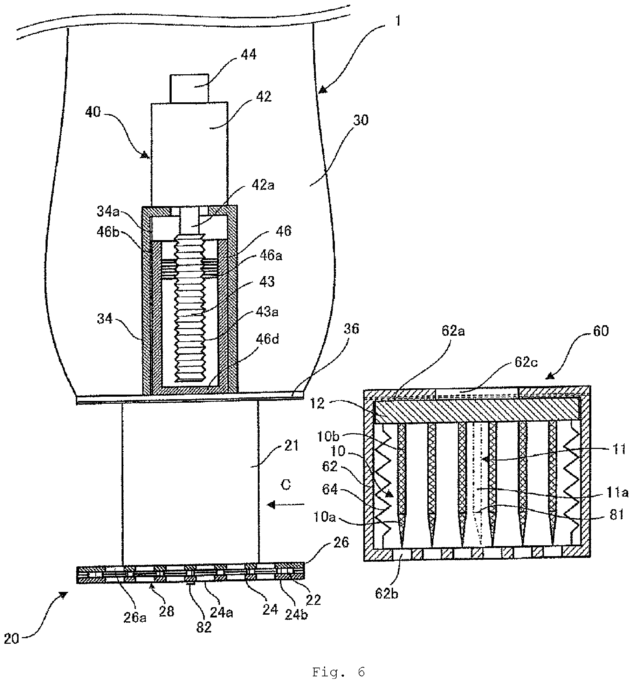

The contacting unit 20 according to the present embodiment is supported to the main body 30 via a support arm 21 (see Fig. 6) to be described later. The contacting unit 20 is attachable to and detachable from the main body 30 or the cartridge 60 via a terminal part 30a (see Fig. 4), and the puncture needle 10, the Peltier elements 22, the temperature sensor 81, and the surface temperature sensor 82 may be electrically connected to the control unit 70. By so doing, it is possible to energize the Peltier elements 22 from the main body 30, and the output of each of the puncture needle 10, the temperature sensor 81, and the surface temperature sensor 82 can be transmitted to the control unit 70. In addition, the terminal part 30a may be provided to the cartridge 60.

The main body 30 includes a cylindrical part 34 provided with an opening 34a in a downward direction in Fig. 5. A hole 34b is formed on a surface opposite to the opening 34a of the cylindrical part 34. The main body 30 and the cylindrical part 34 may be integrally formed or formed as separate bodies.

The drive unit 40 according to the present embodiment includes a drive motor 42 composed of a servo motor and the like, an encoder 44 that detects the number of rotations of the drive motor 42, and a rod 46 that advances and retracts by the rotation of the drive motor 42.

A shaft 43 is connected to a rotating shaft 42a of the drive motor 42, and a screw part 43a is formed on an outer peripheral surface of the shaft 43. In the present embodiment, in order to allow the rotating shaft 42a to penetrate the hole 34b of the cylindrical part 34, the drive motor 42 is arranged outside the cylindrical part 34 and the shaft 43 and the rod 46 are arranged inside the cylindrical part 34.

The rod 46 is formed in a hollow cylindrical shape and is slidably received in the cylindrical part 34 of the main body 30. A nut 46a screwed to the screw part 43a of the shaft 43 is fixed to an inner peripheral surface of the rod 46. On the other hand, a projecting part 46b is provided on an outer peripheral surface of the rod 46 and is engaged with a groove part 34c formed on an inner peripheral surface of the cylindrical part 34, so that the rod 46 is not rotatable.

With the aforementioned configuration of the drive unit 40, the rod 46 can advance in the direction of an arrow B of Fig. 5 by the rotation of the drive motor 42, so that it is possible to control the advance amount of the rod 46 on the basis of the detection of the encoder 44.

With the advance and retraction of the rod 46, the tip part 10a of the puncture needle 10 can penetrate into and retract from the contacting surface 24b. The projection amount of the puncture needle 10 from the lowermost surface of the contacting surface 24b is not particularly limited and for example, can be set to about 0.1 to 10 mm in accordance with target sweat glands. When the puncture needle 10 is arranged in a plural number (for example, about 20 to 40 needles or more), the projection amounts of all the puncture needles 10 preferably fall within the aforementioned numerical value range, and more preferably, the projection amounts of all the puncture needles 10 are approximately equal to one another.

Furthermore, the tip part of the rod 46 is provided with terminals (not illustrated) for supplying power to the respective puncture needles 10 at the time of mounting of the holder 12. The puncture needles 10 are electrically connected to the energy supply unit 2 installed at an exterior. The puncture needle 10 can apply a high-frequency current between the puncture needle 10 and a surface electrode (not illustrated) separately arranged on the epidermis surface of the living body while puncturing the living body. The high-frequency current is applied, so that it is possible to heat the living tissue in the vicinity of the puncture needle 10. When the puncture needle 10 is provided in a plural number, the puncture needles 10 can be configured such that the high-frequency current is applied between two adjacent puncture needles 10. Energization of the puncture needles 10, the contacting unit 20, and the drive unit 40 can be performed by a switch operation of the operating unit 50. However, as the components from the drive unit 40 to the holder 12, any component may be employed if the holder 12 can advance and retract, and for example, when a linear motor is selected as the drive motor 42, components such as the shaft 43 and the like are not always necessary.

As illustrated in Fig. 2, in the cartridge 60, a cartridge body 62 is provided with an engaging groove 62a. The engaging groove 62a is engaged with an engaging rail 36 formed on the main body 30. The cartridge 60 can be attached to the main body 30 in a detachable manner by moving along the engaging rail 36. The fixing of the cartridge 60 to the main body 30 may be performed by fitting of fitting parts (not illustrated) provided in the engaging rail 36 and the engaging groove 62a, or may be performed by holding of the contacting unit 20.

As illustrated in Fig. 5A, the cartridge body 62 is provided therein with the holder 12 that supports the puncture needles 10 and the sensor needle 11. The holder 12 is urged by spring members 64 as an urging unit such that the puncture needles 10 and the sensor needle 11 are retracted into the interior of the cartridge body 62. The cartridge body 62 is provided, on the upper part thereof, with an insertion hole 62c through which a tip part 46d of the rod 46 can pass, and the tip part 46d is configured to be able to press the holder 12 by the advance of the rod 46. The cartridge body 62 is provided, on the lower part thereof, with through holes 62b through which the puncture needles 10 and the sensor needle 11 pass. In order to allow the puncture needles 10 and the sensor needle 11 to be retracted into the interior of the cartridge body 62, the present invention is not limited to the configuration of providing the spring members 64 as an urging unit and other configurations may be employed, for example, the rod 46 and the holder 12 can be allowed to be engaged or disengaged with each other (for example, an engagement state and a disengagement state may be intendedly created by an electromagnet or a mechanical mechanism). In such a case, in a state in which the rod 46 and the holder 12 are engaged with each other, when the rod 46 is pulled up, the holder 12 is also pulled up in interlock with the rod 46, so that the puncture needles 10 are pulled out from the puncture site.

The contacting unit 20 is supported to the main body 30 via the support arm 21, and the cartridge 60 is arranged between the main body 30 and the contacting unit 20. The support arm 21 is flexible and can hold the cartridge 60 between the main body 30 and the contacting unit 20. The support arm 21 is provided therein with a wiring, a signal line, and the like for connecting the contacting unit 20 and the operating unit 50. The through hole 28 of the contacting unit 20 is formed to be aligned with the through hole 62b of the cartridge 60. The shapes of the through hole 28 and the through hole 62b are not particularly limited. The cartridge 60 may be formed of a mesh plate, a porous plate, and the like or may be formed by cutting off a part of a sidewall in order to improve heat dissipation from the heat dissipation block 26.

The control unit 70 controls the driving of the drive unit 40 by operating the operating unit 50, thereby allowing the puncture needle 10 to be projected and retracted. Preferably, the control unit 70 controls the driving of the drive unit 40 such that the tip part 10a of the puncture needle 10 stops at each heating depth position stored in advance. In addition, the control unit 70 may be embedded in the main body 30 or configured externally by a smart phone, a personal computer, and the like.

Furthermore, the control unit 70 energizes the contacting unit 20 to cool the contacting surface 24b that comes into contact with the epidermis and the like. During the cooling, preferably, the temperatures of the interior and the surface of the living body are measured by the puncture site temperature sensor 81 and the surface temperature sensor 82, and the control unit 70 controls the cooling temperature of the contacting surface 24b in accordance with the measured values.

Moreover, the control unit 70 sequentially supplies energy including thermal energy to a target tissue such as sweat glands from the tip part 10a at each heating depth position. The energy including thermal energy is supplied from the energy supply unit 2. The sequential supply means that the energy including thermal energy is supplied from the tip part 10a of the puncture needle 10 while sequentially increasing a distance of the tip part 10a of the puncture needle 10 from the epidermis and the like step by step. By so doing, it is possible to perform uniform heating along the depth direction of the puncture site and to efficiently destroy the entire sweat glands present in this area.

The control unit 70 may control the amount of the energy, which includes thermal energy and is supplied from the energy supply unit 2, in accordance with the temperature measured by the temperature sensor 81 for measuring the temperature of the puncture site. That is, since the amount of the energy including thermal energy required for destroying a target sweat gland is determined in accordance with the kind of the target sweat gland, it is possible to control the energy supply unit 2 for that purpose. For example, it is possible to keep a state, in which the temperature measured by the temperature sensor 81 is equal to or more than a predetermined temperature, for a predetermined time. As described above, the amount of the energy including thermal energy is controlled, so that it is possible to precisely heat the puncture site and efficiently destroy sweat glands.

The energy including thermal energy supplied from the puncture needle 10 may be changed in accordance with a depth interval, or large energy including thermal energy may be supplied as the depth interval is large. The energy including thermal energy may be supplied by high frequency, radio wave, microwave, laser, and the like. In these cases, the amount of the energy supplied from the energy supply unit 2 differs depending on a target sweat gland, and the amount of required energy may be obtained in advance by an experiment and the like. This is the same even in the case of the thermal energy.

In the surface of the epidermis, since the periphery of the puncture site is cooled by the close contact of the contacting surface 24b, it is possible to prevent thermal burn and reduce pain during puncture and thermal energy supply. Anesthetic cream may be applied in advance on the surface of the epidermis that closely contacts with the contacting surface 24b. In a configuration in which the contacting surface 24b injects cooling gas to the epidermis surface, the injection of the cooling gas is started immediately before the puncture needle 10 punctures the epidermis surface, so that it is possible to obtain good thermal burn prevention effect and analgesic effect.

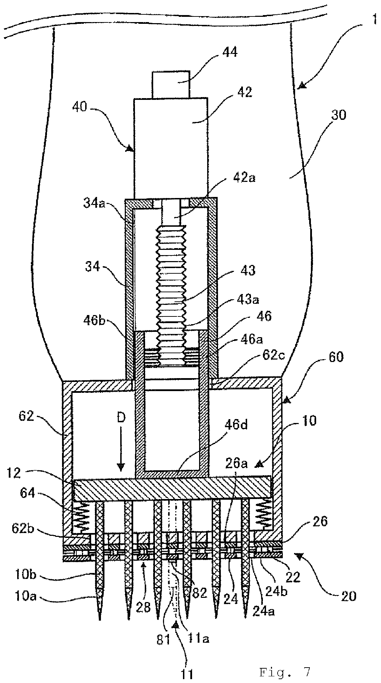

Next, an operation of the puncture device 1 having the aforementioned configuration will be described using Fig. 6 and Fig. 7. Fig. 6 is a view illustrating a state before the cartridge 60 is attached to the main body 30 of the puncture device 1 according to the embodiment according to the present invention. Fig. 7 is a view illustrating a state in which the drive unit 40 of the puncture device 1 according to the embodiment according to the present invention is operated.

First, the cartridge 60 including the puncture needle 10 is attached to the main body 30. The cartridge 60 can be attached to the main body 30 by sliding along the engaging rail 36 in the direction of an arrow C of Fig. 6.

In the state in which the cartridge 60 has been attached, the cartridge 60 may be held by the contacting unit 20 and the main body 30 such that the insertion hole 62c of the cartridge 60 is arranged at a position corresponding to the tip part 46d of the rod 46. Furthermore, a convex part and a concave part (not illustrated) may be provided in the cartridge 60 and the main body 30 so as to be engaged with each other.

When the cartridge 60 is attached to the main body 30, a terminal part (not illustrated) of the main body 30 is engaged with a terminal part (not illustrated) of the cartridge 60 to energize the temperature sensor 81 for measuring the temperatures of the puncture needle 10 and the puncture site.

When the operating unit 50 illustrated in Fig. 1 is operated, the drive motor 42 of the drive unit 40 rotates. When the drive motor 42 rotates, the rotating shaft 42a and the shaft 43 rotate. The nut 46a is screwed to the shaft 43, and the rod 46 is not rotatable because the projecting part 46b is engaged with the groove part 34c. Consequently, as illustrated in Fig. 7, the rod 46 moves in the direction of an arrow D in accordance with the rotation amount of the drive motor 42, so that each puncture needle 10 gradually projects from the contacting surface 24b.

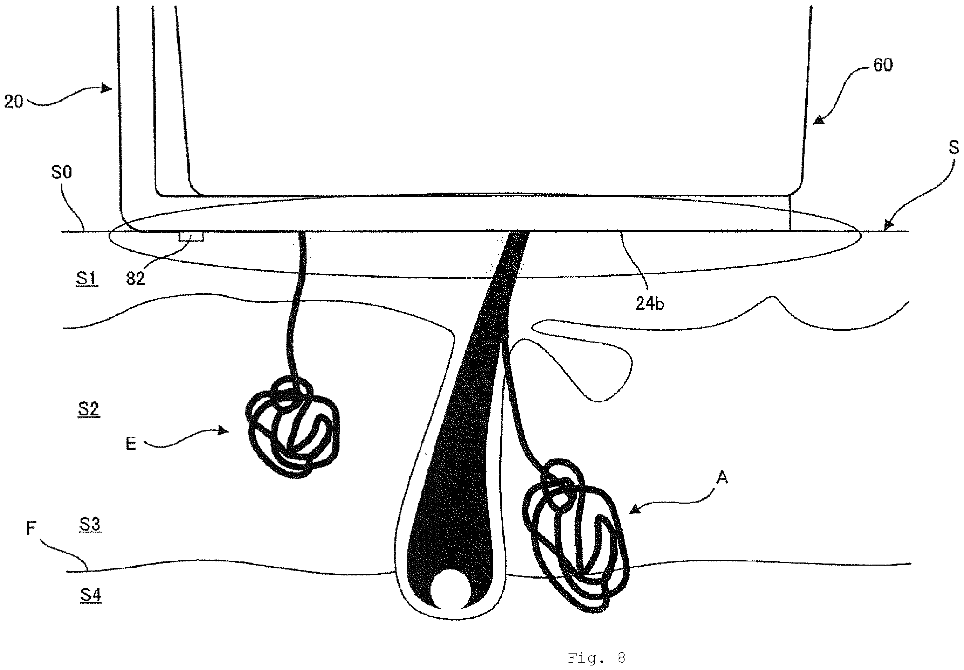

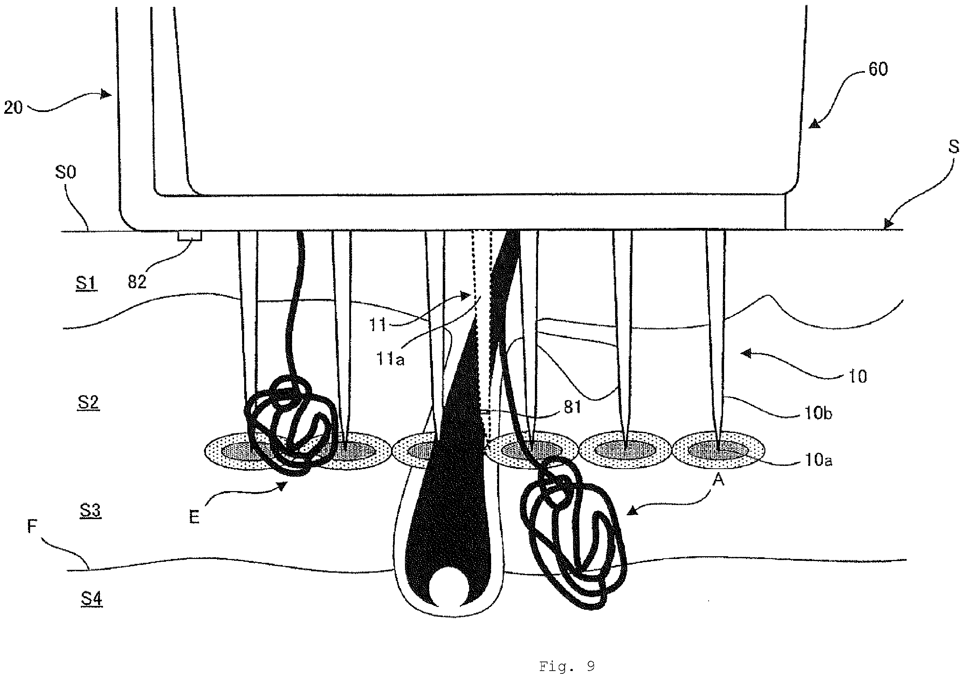

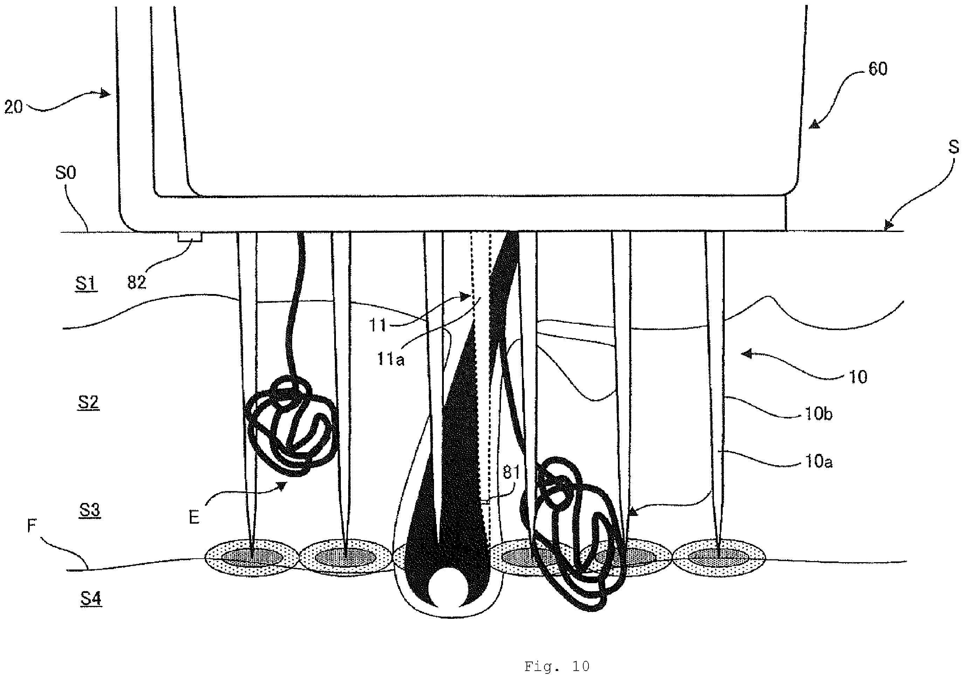

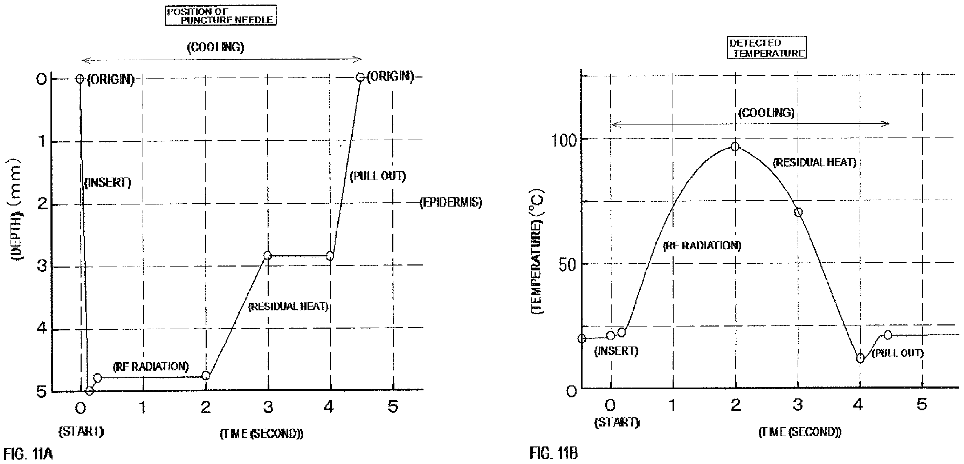

Next, an example of using the puncture device 1 according to the aforementioned embodiment for a living body S to destroy sweat glands E and A will be described using Fig. 8 to Fig. 11. Fig. 8 is a view illustrating a state before the puncture needle 10 of the puncture device 1 according to the embodiment according to the present invention is projected. Fig. 9 is a view illustrating a state in which the puncture needle of the puncture device according to the embodiment of the present invention is projected. Fig. 10 is a view illustrating a state in which the puncture needle of the puncture device according to the embodiment of the present invention has reached a membrane. Fig. 11A is a graph illustrating change over time of the tip position of the puncture needle when the puncture device according to the embodiment of the present invention is operated, and Fig. 11B is a graph illustrating change over time of a temperature detected by the sensor needle in a similar manner.

First, the tissue under the skin will be described. An eccrine gland (E gland) is present in the body other than lips, and an apocrine gland (A gland) is distributed from the armpits to the chest, the ears, the areola, the navel, and the lower parts of the torso, and the range of treatment differs depending on patients. Both the A gland and the E gland are present below the dermis tissue (part deeper than the dermis). For example, in the average of 60 cases for Japanese, the size of the A gland is 0.48 mm, the shallowest part is 0.812 mm in the subcutaneous area, the deepest part is 2.591 mm in the subcutaneous area, and the center of the A gland is 1.51 mm in the subcutaneous area. In a similar manner, on average, the size of the E gland is 0.57 mm, and it is elongated with a long axis (vertical axis) of 1.47 mm and a short axis (horizontal axis) of 0.57 mm, the shallowest part is 0.72 mm, the deepest part is 3.01 mm, and the center of the E gland is 1.74 mm in the subcutaneous area. However, since this is the average of 60 cases for Japanese, it may be changed by increasing the number of cases or it does not always match by racial differences and the like.

In the following puncture device 1 according to the embodiment according to the present invention, energy emitted from the puncture needle 10 is thermal energy; however, in the puncture device according to the present invention, the energy emitted from the puncture needle is not limited to the thermal energy.

As illustrated in Fig. 8, the contacting surface 24b of the contacting unit 20 of the puncture device 1 is first brought into close contact with an epidermis surface SO of an epidermis and the like S1. After the contacting surface 24b is brought into close contact with the epidermis surface SO, the epidermis surface SO is cooled by the cooling plate 24 of the contacting unit 20. During the cooling, it is sufficient if the control unit 70 adjusts the temperature of the cooling plate 24 in accordance with a temperature measured by the surface temperature sensor 82.

Next, the drive motor 42 of the drive unit 40 is rotated as illustrated in Fig. 7 by operating the operating unit 50 illustrated in Fig. 1. When the drive motor 42 of the drive unit 40 is rotated, each puncture needle 10 gradually projects from the contacting surface 24b as illustrated in Fig. 9. As the projection amount of the puncture needle 10 from the contacting surface 24b becomes large, the tip of the puncture needle 10 enters a dermis S2 from the epidermis and the like S1.

At this point of time, thermal energy is supplied to a target tissue from the tip part 10a of the puncture needle 10. The supply amount of the thermal energy may be appropriately changed by the control unit 70 in accordance with a temperature measured by the temperature sensor 81 for measuring the temperature of a puncture site. It is sufficient if the cooling temperature of the cooling plate 24 is adjusted by the control unit 70 in accordance with the temperature measured by the temperature sensor 81. Furthermore, the supply of the thermal energy to the target tissue from the tip part 10a of the puncture needle 10 may be stopped depending on the temperature measured by the temperature sensor 81. In addition, in Fig. 9 and Fig. 10, the center part of a double elliptic part drawn in the vicinity of the tip part 10a of the puncture needle 10 is a part to which high thermal energy is applied, and the outer circumferential part is a part to which lower thermal energy is applied. The thermal energy applied to a further outer circumferential side of the double elliptic part can be substantially ignored.

As the projection amount of the puncture needle 10 from the contacting surface 24b becomes large, the tip of the puncture needle 10 enters a subcutaneous tissue S3 via the dermis S2 from the epidermis and the like S1. When the tip of the puncture needle 10 reaches a membrane F present between the subcutaneous tissue S3 and a muscle layer S4, the puncture needle 10 is not able to penetrate the membrane F and the puncture depth of the puncture needle 10 is constantly maintained. Since the puncture needle 10 is configured not to penetrate the membrane F, the tip part 10a of the puncture needle 10 may be formed in the shape of the blunt needle (the non-beveled needle having no blade surface on the tip thereof) as described above or the thickness of the puncture needle 10 may be adjusted in accordance with the performance of the drive motor 42 and the like of the drive unit 40, and a pressure sensor and the like capable of measuring the pressing force of the puncture needle 10 may be provided and the control device 70 may control the rotation of the drive motor 42 of the drive unit 40 and appropriately set the pressing force of the puncture needle 10 in accordance with a detection result of the pressure sensor and the like. Furthermore, a configuration of understanding a depth area in advance, which will be described later, may be added to the adjustment and the setting.

When the puncture depth of the puncture needle 10 is constantly maintained for a predetermined time, the control device 70 stops the drive unit 40. The projection amount of the puncture needle 10 at this time may be calculated from a value of the encoder 44 and stored in a memory as maximum depth information (excluding a case where the projection amount of the puncture needle 10 from the contacting surface 24b is a prescribed maximum projection amount). Thereafter, the control device 70 moves the puncture needle 10 in the withdrawal direction from the puncture site by reversely rotating the drive motor 42, so that the tip of the puncture needle 10 is buried above the contacting surface 24b. Preferably, the thickness or the pressing force of the puncture needle 10 is appropriately set, similarly to the membrane F, such that the tip part 10a does not reach the muscle layer S4 and reliably remains in the subcutaneous tissue S3.

The maximum depth information acquired as described above, that is, information on the depth to the membrane F corresponds to a depth area where there is a sweat gland such as an apocrine gland at the puncture site. Thus, in addition to the understanding of the depth area in advance, by understanding a relative position (a position in a plane direction perpendicular to the projection direction of the puncture needle 10 (the same applies below)) of the contacting unit 20 relative to the epidermis of the living body, when treatment is repeated with respect to the same epidermis surface SO (or the epidermis and the like S1), a user may select an automatic mode by operating the operating unit 50 and set efficient treatment to be performed.

That is, when the automatic mode is set, the control device 70 first sets a plurality of heating depth positions on the basis of the maximum depth information. The heating depth positions, for example, can be obtained from a depth interval determined by comparing a value of a maximum depth with a prescribed reference value.

A specific example will be described. When the maximum depth is 4 mm or less, the depth interval can be set to 0.4 mm and the heating depth positions can be set as respective depth positions of 0.4 mm, 0.8 mm, 1.2 mm, ... from the epidermis surface. When the maximum depth exceeds 4 mm, the depth interval can be set to 0.6 mm and the heating depth positions can be set as respective depth positions of 0.6 mm, 1.2 mm, 1.8 mm, ... from the epidermis surface SO.

The reference value of the maximum depth and the depth interval are not limited to the aforementioned values, and a plurality of reference values and a plurality of depth intervals can be set. Furthermore, the depth interval does not always need to be constant, and may be set to be gradually decreased (or increased) with an increase in the puncture depth. The maximum depth information and the heating depth position may be output to a monitor of the control device 70 so that a user can understand the maximum depth information and the heating depth position together with the relative position of the contacting unit 20 relative to the epidermis of the living body.

After the heating depth positions are determined as described above, the control device 70 may energize the contacting unit 20 to cool the epidermis surface SO with which the contacting surface 24b closely contacts, and control the driving of the drive unit 40 such that the tip part 10a of the puncture needle 10 stops at each heating depth position, so that thermal energy may be supplied to the target tissue from the tip part 10a at each heating depth position. By repeating these steps, it is possible to perform uniform heating along the depth direction of the puncture site and to efficiently and reliably destroy the entire sweat glands present in this area.

For example, since the thermal energy is supplied to the depth position of 0.4 mm from the epidermis surface SO, the tip part 10a of the puncture needle 10 sequentially punctures to the depth position of 0.4 mm in the entire area of the epidermis surface SO to be treated and is energized to treat the epidermis surface SO. Next, since the thermal energy is supplied to the depth position of 0.8 mm from the epidermis surface SO, the tip part 10a of the entire puncture needle 10 punctures to the depth position of 0.8 mm and is energized to treat the epidermis surface SO. By repeating these steps, it is possible to perform uniform heating along the depth direction of the puncture site.

The thermal energy supplied from the puncture needle 10 may be changed in accordance with the depth interval, and large thermal energy may be supplied as the depth interval is large. This is based on the consideration that the amount of required thermal energy differs because a target sweat gland differs in accordance with the depth interval. The supply of the thermal energy or the energy may be performed using various kinds including high frequency, radio wave, microwave, laser, and the like.

In the puncture device 1 according to the embodiment of the present invention, an alternating current (hereinafter, referred to as "RF current" in some cases) selected from 100 Hz to 3 THz is caused to flow, for example, between a large surface electrode (not illustrated) attached to the femoral region and the puncture needle 10, so that heat is generated in the vicinity of the tip part of the puncture needle 10. This heat generation is due to the fact that since the density of the RF current flowing through the tip part of the puncture needle 10 becomes large, heat is generated in the vicinity of the tip part of the puncture needle 10. As the RF current, a low-frequency alternating current of about 100 Hz to 1000 Hz is preferably used from the standpoint of stability and efficiency. An applied voltage is about 100 to 400 V, a high voltage is employed when an application time is short, and a low voltage is employed when the application time is long.

The desired temperature is 50°C or more, and, for example, 50°C to 150°C is employed. This temperature can be set by the control unit 70. By this setting, for example, the temperature can be set to 50°C to 120°C, for example, 50°C to 100°C or 70°C or more. In order to reach the set temperature, a predetermined RF current is supplied from the tip part of the puncture needle 10. Although not particularly limited, the amount of the RF current can be set by the applied voltage of the RF wave, and specifically, the peak-to-peak voltage of the RF wave can be set as a target voltage.

Even though the temperature exceeds 100°C, when it is a short time, there is no concern of burn. As the heating time, 1 to 10 seconds are empirically employed, and 2 seconds or more are preferable for reliable treatment. After the RF current flows through the puncture needle 10, it takes about one second to reach a predetermined temperature, and then the puncture needle 10 is heated to reach 50°C or more for a predetermined time. Then, when the energization stops, the temperature decreases. The longer the energization time, the greater the destruction effect of the sweat glands. However, when the energization time is long, since the burden on a physician holding the puncture needle 10 increases and also it is easy to burn, it is better to keep the energization time as short as possible. For example, when the puncture needle 10 is provided at an interval of 2 mm, if the tip of the puncture needle 10 is energized to reach 100°C, the temperature of an intermediate part of the puncture needle 10 can be set to 50°C or more, for example, about 70°C.

Fig. 11A illustrates an example of change over time of the tip position of the puncture needle 10 when the puncture device 1 according to the embodiment of the present invention is operated, and Fig. 11B illustrates an example of change over time of a temperature detected by the sensor needle 11 in a similar manner. The origin and the start temperature in Fig. 11A and Fig. 11B correspond to the state illustrated in Fig. 8. Fig. 11A and Fig. 11B are examples of the operation of the puncture device 1, but the operation of the puncture device 1 is not limited thereto.

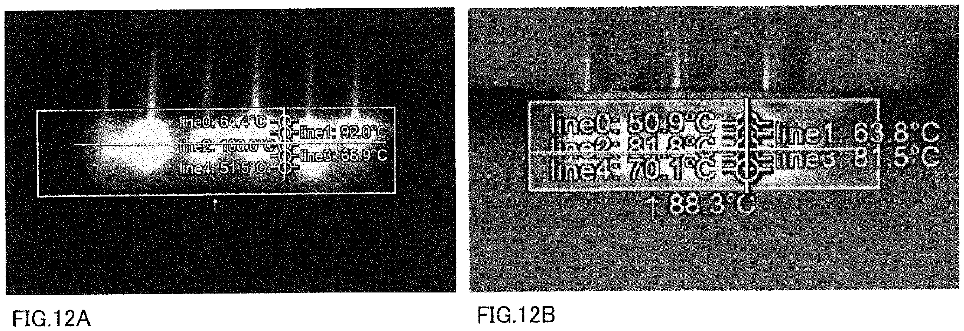

Fig. 12A illustrates a temperature distribution measurement result at a predetermined point of time when the RF current flows through the puncture device according to the embodiment of the present invention, without using the cooling plate 24, and Fig. 12B illustrates a temperature distribution measurement result at a predetermined point of time when the RF current flows through the puncture device according to the embodiment of the present invention, in a similar manner by using the same cooling plate 24. Fig. 12A and Fig. 12B illustrate predetermined moments during the heating time and both times do not match with each other. In Fig. 12A and Fig. 12B, the puncture site is a creamy medium imitating the human skin.

Temperature measurement points of line 0 to line 4 are set from a shallow part toward a deep part in the depth direction and the intermediate line 2 corresponds approximately to the depth of the tip of the puncture needle 10.

In any cases of Fig. 12A and Fig. 12B, it can be seen that the temperature of the line 2 corresponding approximately to the tip of the puncture needle 10 is maximum. Since the depth position of the temperature sensor 81 approximately coincides with the depth position of the tip of the puncture needle 10, the temperature sensor 81 can detect the temperature of a part having the maximum temperature at the position in the depth direction. Since the temperature sensor 81 feeds back the maximum temperature in the depth direction, it is possible to perform appropriate temperature control. However, the temperature detected by the temperature sensor 81 is not limited only to the temperature of the depth position corresponding to the tip of the puncture needle 10, and for example, it is also possible to detect the temperature of a position shallower or deeper than the depth position corresponding to the tip of the puncture needle 10. Furthermore, for example, in addition to the temperature of the depth position corresponding to the tip of the puncture needle 10, it is also possible to detect the temperatures of a plurality of positions at different depths. In order to detect the temperatures of the depth positions, a plurality of sensor needles 11 can be used, but a plurality of temperature sensors 81 are provided in the length direction of a single sensor needle 11, so that the temperatures of the depth positions can be detected by the single sensor needle 11. Moreover, the temperatures of the depth positions are fed back, so that it is possible to more appropriately control a temperature distribution in the depth direction.

Furthermore, the temperature of the line 0 near the epidermis surface SO is relatively low in the case of Fig. 12B in which the cooling plate 24 is provided as compared with the case of Fig. 12A in which there is no cooling plate 24, and it can be seen that the cooling effect for the epidermis surface SO is obtained by the cooling plate 24. That is, in Fig. 12A, when the line 3 is 68.9°C and the line 4 is 51.5°C, the line 0 is 64.4°C, but in Fig. 12B, when the line 3 is 81.5°C and the line 4 is 70.1°C, the line 0 is 50.9°C.

In the epidermis surface SO, since the periphery of the puncture site is cooled by the close contact of the contacting surface 24b, it is possible to prevent thermal burn and reduce pain during puncture and thermal energy supply. In addition, anesthetic cream may be applied in advance on the epidermis surface SO that closely contacts with the contacting surface 24b. Alternatively, the contacting surface 24b may be configured to inject cooling gas to the epidermis surface SO without applying the anesthetic cream or in addition to the application of the anesthetic cream. In such a case, the injection of the cooling gas is started immediately before the puncture needle 10 punctures the epidermis surface SO, so that it is possible to obtain good thermal burn prevention effect and analgesic effect. Moreover, even during the puncturing period, the cooling gas is injected to the epidermis surface SO from the opening 24a, so that it is possible to further maintain the cooling effect and thus to obtain better thermal burn prevention effect and analgesic effect.

After the thermal energy is supplied, the puncture needle 10 is lifted by the operation of the drive unit 40 and is received in the cartridge 60 again. By so doing, the treatment of the target tissue is ended. In a case where treatment for a different living body is continued, and the like, the cartridge 60 is replaced with a new one and then the treatment can be started. As described above, the cartridge 60 is configured to be detachable and replaceable, so that it is not necessary to sterilize the puncture needle 10 every time and it is possible to perform quick treatment. In addition, the contacting unit 20 can also be removed from the main body 30 and can be replaced with a new one, but may be fixed.

The puncture device 1 may acquire in advance a depth area, where sweat glands are likely to be present at a puncture site, as maximum depth information prior to the request of thermal energy. The maximum depth information is acquired in advance, so that it is possible to efficiently supply the thermal energy to the sweat glands and quickly and accurately perform treatment for suppressing excessive sweating. In the present invention, the suppression of the sweating aims to reduce an odor due to sweating even though the amount of the sweating is small (for example, hircismus (tragomaschalia) treatment), in addition to reducing the amount of the sweating itself (for example, hyperhidrosis treatment).

So far, the puncture device 1 according to the present embodiment includes the puncture needle 10 that punctures the puncture site of the living body and supplies thermal energy, the sensor needle 11 having the temperature sensor 81 that measures the temperature of the puncture site, the holder 12 that holds the puncture needle 10 and the sensor needle 11, the cartridge 60 that receives the puncture needle 10, the sensor needle 11, and the holder 12, the main body 30 to which the cartridge 60 is attachable, the contacting unit 20 provided with the through holes 28 penetrating the front and back surfaces thereof and provided on the surface side thereof with the contacting surface 24b with the surface of the puncture site, and the drive unit 40 that moves the holder 12 such that the puncture needle 10 and the sensor needle 11 are projected and retracted from the contacting surface 24b via the through holes 28. Consequently, it is possible to understand to what degree the living body is being heated and to suppress the living body from being excessively heated and insufficiently heated.

In the puncture device 1 according to the present embodiment, the contacting unit 20 includes the cooling part. Consequently, it is possible to suppress the formation of a burn and a scab by cooling the puncture site of the living body.

The puncture device 1 according to the present embodiment includes the control unit 70 that controls the cooling part in accordance with the temperature measured by the temperature sensor 81 that measures the temperature of the puncture site. Consequently, it is possible to suppress the puncture site of the living body from being excessively heated and to cool the puncture site.

The puncture device 1 according to the present embodiment has the surface temperature sensor 82 that measures the temperature of the surface of the puncture site on the surface of the contacting surface 24b. Consequently, it is possible to understand the temperature of the surface of the puncture site of the living body and to suppress the puncture site of the living body from being excessively heated.

The puncture device 1 according to the present embodiment includes the control unit 70 that controls the cooling part in accordance with the temperature measured by the surface temperature sensor 82. Consequently, it is possible to suppress the surface of the puncture site of the living body and the puncture site from being excessively heated and to cool the puncture site.

The cartridge 60 for the puncture device according to the present embodiment includes the puncture needle 10 that punctures the puncture site of the living body and supplies the thermal energy, the sensor needle 11 having the temperature sensor 81 that measures the temperature of the puncture site, and the holder 12 that holds the puncture needle 10 and the sensor needle 11. Consequently, it is possible to easily replace the cartridge 60 from the main body 30.

The present invention is not limited by this embodiment, and various variations and modifications will not deviate from the scope of the present invention.

### Reference Signs List

- 1: puncture device
- 2: energy supply unit
- 10: puncture needle
- 11: sensor needle
- 11a: hollow needle
- 12: holder
- 20: contacting unit
- 21: support arm
- 22: Peltier element
- 24: cooling plate
- 24a: opening
- 24b: contacting surface (cooling surface)
- 26: heat dissipation block
- 26a: opening
- 28: through hole
- 30: main body
- 34: cylindrical part
- 34a: opening
- 34b: hole
- 34c: groove part
- 36: engaging rail
- 40: drive unit
- 42: drive motor
- 43: shaft
- 44: encoder
- 46: rod
- 46a: nut
- 46b: projecting part
- 46d: tip part
- 50: operating unit
- 60: cartridge
- 62: cartridge body
- 62a: engaging groove
- 62b: through hole
- 62c: insertion hole
- 64: spring member
- 70: control unit
- 81: temperature sensor
- 81a: SUS hollow needle
- 81b: insulating coating
- 81c: lead wire
- 82: surface temperature sensor

## Claims

1. A puncture device comprising:
a puncture needle configured to puncture a puncture site of a living body and supply energy to the puncture site;
a holder configured to hold the puncture needle;
a cartridge configured to receive the holder so as to be slidable and having an opening through which the puncture needle is projected;
a main body to which the cartridge is detachably attached;
a contacting unit having a through hole through which the puncture needle is projected to the puncture site of the living body and having a cooling member that is able to come into contact with the puncture site;
a sensor needle in which a temperature sensor is arranged; and
a control unit, wherein
the sensor needle is installed in parallel with the puncture needle and is held by the holder so as to project to the puncture site of the living body via the opening formed in the cartridge and the through hole formed in the contacting unit, and
the control unit controls the energy supplied to the puncture needle, in accordance with output of the temperature sensor.

2. The puncture device according to claim 1, wherein the control unit supplies the energy to the puncture needle such that an output temperature of the temperature sensor is 50°C or more and 150°C or less.

3. The puncture device according to claim 1, wherein the control unit supplies an alternating current to the puncture needle.

4. The puncture device according to claim 1, wherein the control unit is configured to be able to supply an alternating current to the puncture needle for a prescribed time.

5. The puncture device according to claim 4, wherein the prescribed time is a time between one second and 20 seconds.

6. The puncture device according to claim 1, wherein a surface of the puncture needle is coated with an insulating material in a state in which a tip side of the puncture needle is exposed.

7. The puncture device according to claim 6, wherein an exposure length of the tip side of the puncture needle is 0.1 to 2.0 mm.

8. The puncture device according to claim 6 or 7, wherein the insulating material is made by glass coating or resin coating.

9. The puncture device according to claim 1, wherein a surface temperature sensor is provided on a side of the contacting unit, the side facing the puncture site, to measure a temperature of a surface of the puncture site, and
the control unit controls a temperature of the cooling member in accordance with output of the surface temperature sensor.

10. A cartridge for a puncture device, the cartridge comprising:
a puncture needle configured to puncture a puncture site of a living body and supply energy to the puncture site;
a holder configured to hold the puncture needle;
a cartridge configured to receive the holder so as to be slidable and having an opening through which the puncture needle is projected; and
a sensor needle in which a temperature sensor is arranged, wherein
the sensor needle is installed in parallel with the puncture needle and is held by the holder so as to project via an opening formed in the cartridge.

11. The cartridge for a puncture device according to claim 10, wherein a surface of the puncture needle is coated with an insulating material in a state in which a tip side of the puncture needle is exposed.

12. The cartridge for a puncture device according to claim 11, wherein an exposure length of the tip side of the puncture needle is 0.1 to 2.0 mm.

13. The cartridge for a puncture device according to claim 11 or 12, wherein the insulating material is made by glass coating or resin coating.
